# EUROPEAN PATENT SPECIFICATION

(11) **EP 2 381 961 B1**
(45) Date of publication and mention of the grant of the patent: **14.09.2016**
(21) Application number: 09804035.5
(22) Date of filing: 24.12.2009
(51) Int. Cl.: A61K 47/10, A61K 47/20, A61K 47/26, A61K 47/32, A61K 9/00, A61K 31/505

(54) **IMPLANTABLE DEVICES FOR TREATING HIV**
IMPLANTIERBARE VORRICHTUNGEN ZUR BEHANDLUNG VON HIV
DISPOSITIFS IMPLANTABLES POUR LE TRAITEMENT DU VIH

(30) Priority: 24.12.2008 US 140694 P
(43) Date of publication of application: 02.11.2011
(73) Proprietor: Janssen Sciences Ireland UC, Little Island, County Cork (IE)
(72) Inventor: SCHACHTER, Deborah M., Edison NJ 08817 (US); ZHANG, Qiang, Annandale New Jersey 08801 (US); BAERT, Lieven Elvire Colette, 8200 Brugge 2 (BE); CUI, Han, Basking Ridge New Jersey 07920 (US)
(74) Representative: Vervoort, Liesbeth
(86) International application number: PCT/EP2009/067933
(87) International publication number: WO 2010/072844

(56) References cited:
- WO-A2-2007/082922
- SMITH A ET AL: "An assessment of the use of implanon(R) in three community services" JOURNAL OF FAMILY PLANNING AND REPRODUCTIVE HEALTH CARE 200210 GB LNKD- DOI:10.1783/147118902101196540, vol. 28, no. 4, October 2002 (2002-10), pages 193-196, XP002583682 ISSN: 1471-1893

## Description

### Field of the Invention

The present invention relates to an implantable device of the NNRTI TMC278 as defined by the claims, which can be used in the prevention and suppression of HIV infection.

### Background of the Invention

The treatment of Human Immunodeficiency Virus (HIV) infection, which is causative to the acquired immunodeficiency syndrome (AIDS), remains a major medical challenge. The HIV is able to evade immunological pressure, to adapt to a variety of cell types and growth conditions and to develop resistance against currently available drug therapies. The current standard therapy involves the administration of at least three agents selected from nucleoside reverse transcriptase inhibitors (NRTIs), non-nucleoside reverse transcriptase inhibitors (NNRTIs), HIV-protease inhibitors (PIs), and the more recent fusion inhibitors. In countries with broad access to effective antiretroviral therapy (ART) the clinical benefits have been dramatic. Far fewer HIV-infected people progress to AIDS. However, adherence to ART has emerged as both the major determinant and the Achilles heel of this success. Antiretroviral adherence is the second strongest predictor of progression to AIDS and death after CD4 count. Incomplete adherence to ART is common in all groups of treated individuals, despite the fact that long-term viral suppression requires near-perfect adherence. The resulting virologic failure diminishes the potential for long-term clinical success. Drug-resistant strains of HIV selected through ongoing replication in the presence of ART also can be transmitted to uninfected or drug-naive patients, leaving them with fewer treatment options.
Although adherence is important for all of the drug classes in ART, adherence is especially important for the NNRTI class. The balance between viral suppression and resistance for this class of drugs is especially precarious. This precariousness is the result of the low genetic barrier of the NNRTI class of drugs relative to protease inhibitors. While resistance to protease inhibitors requires multiple mutations, where each mutation can reduce enzymatic efficiency and viral fitness, acquisition of only a single mutation appears to confer cross-class resistance to all three available agents. Therefore, if HIV does escape NNRTI control, resistant virus emerges swiftly.

Currently, the available NNRTI therapies are all oral therapies. Maintaining the adherence, which is necessary to prevent resistance, is therefore challenging. The regimen that requires this high level of compliance requires that in addition to the large number of pills ingested daily, the timing of the pills must be extremely regular. The regularity of the dosing ensures that the concentration of the drug in the plasma is maintained and does not drop to below sub-optimal levels. This is very difficult to maintain on a daily basis for a lifetime but the consequences to not adhering to the regimen can be fatal.

TMC278, otherwise known as 4-[[4-[[4-(2-cyanoethenyl)-2,6-dimethylphenyl}-amino]-2-pyrimidinyl]-amino]-benzonitrile and having the generic name rilpivirine, is an NNRTI currently under clinical development. This compound as well as its preparation is described in WO 2003/16306.

One way of overcoming the problems associated with anti-HIV drug adherence is by providing long-acting drug therapy whereby the effective drug plasma levels are maintained during long periods of time, without frequent administrations.
WO 2006/106103 describes the use of parenteral formulations of TMC278 for the long-term prevention of HIV infection, while WO 2007/082922 describes the use of parenteral formulations of TMC278 for the long-term suppression of HIV infection. WO 2007/082922 in turn describes the use of micro- or nanoparticulate formulations for as well the long-term prevention as suppression of HIV infection. The formulations described in these references provided long-lasting effective drug plasma levels.

Clinical studies with TMC278 unveiled some side effects including nausea, dizziness, abnormal dreams, dyspepsia, asthenia, skin rashes, somnolence and vertigo, although these occurred less frequently than with the NNRTIs that are on the market. In particular rashes are a side effect frequently encountered with existing NNRTIs, usually developing within the first 3 - 4 weeks of treatment. If these become sufficiently severe the medication must be terminated. Termination of the medication is easy to achieve for oral dosage forms. However, the nature of the long-lasting formulations described in the references of the previous paragraph, is such that it would not be possible to retrieve them should the injected patient demonstrate any adverse reaction to the therapy.

Hence there is a need for HIV inhibitory therapy that avoids a high pill burden, does not require frequent dosing, but is removable in the case of adverse drug reactions. It has been found that implants comprising a degradable polymer and TMC278 provide sustained release of this active ingredient during long periods of time. In order to be removable, such implants preferably have to be made in one piece and additionally have to be of a certain size in order to contain a sufficient amount of active ingredient as to exert a long-lasting therapeutic effect. A problem associated with such implants is that initially the drug release is insufficient because of the time needed for the body fluids to penetrate the implant. It now has been found that the addition of specific agents overcomes this initial drop in the release of TMC278 from the implant.

### Brief Description of Figures

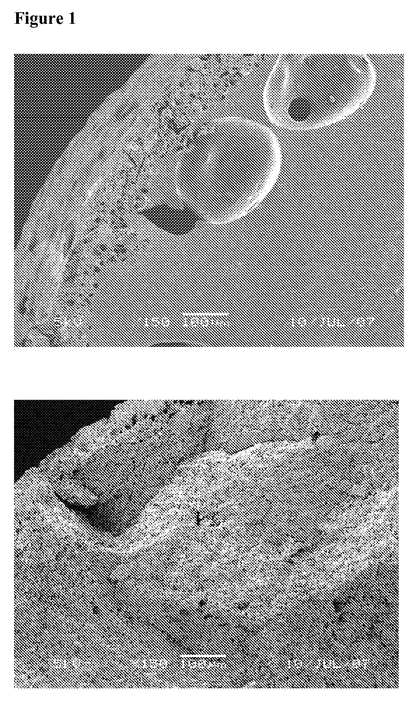

Figure 1: Scanning electron micrographs (SEMs) of PLGA 50/50 rods containing 60% TMC278 (left) without DMSO and (right) with 10% (w/w) DMSO after 4 weeks incubation in PBS at 37°C.
Figure 2: (left) Differential scanning calorimetry thermogram (first heat) of recrystallized TMC278 dispersed in PLGA, with (right) a thermogram (first heat) of TMC278 dispersed in DMSO/ PLGA.
Figure 3: SEM micrographs of (left) TMC278 crystals after re-crystallization and (right) before recrystallization.

### Description of the Invention

This invention concerns an implantable device comprising a biocompatible, biodegradable polymer mixed with TMC278 and with one or more release-enhancing agents selected from the group consisting of poloxamers, polysorbates, and a combination of dimethyl sulfoxide (DMSO) and poly(vinyl pyrrolidone)(PVP) as defined by the claims

The implantable device in particular is a one-piece device. In one embodiment the weight of the device is equal or greater than 100 mg, or is equal or greater than 200 mg, or is equal or greater than 400 mg, or is equal or greater than 500 mg, or is equal or greater than 800 mg, or is equal or greater than 1000 mg, or is equal or greater than 1200 mg, or is equal or greater than 1200 mg. Too large devices are not practicable, an upper limit may be about 2 g; or about 1.5 g.

The percent by weight of TMC278 in the implantable device of the disclosure may be from about 10% to about 80%, from about 10% to about 70%, or from about 20% to about 65%, or from about 25% to about 60% or from about 40% to about 60%, or from about 50% to about 80%, or from about 50% to about 60%. In one embodiment the device contains from about 50% to about 70%, or from about 55% to about 65%, for example about 60% of TMC278. The higher loadings of TMC278, such as in the above ranges starting at about 50%, are preferred where less frequent administrations are desired, this to keep the devices sufficiently compact for convenience of administration and for the comfort of the patient.

The concentration of the release-enhancing agent in the implantable devices of this invention may be in the range from about 1% to about 40% , or of about 5% to about 35%, or of about 10% to about 40%, or of about 15% to about 30%, e.g. about 20% or about 30%. In other embodiments the concentration of the release-enhancing agent in the implantable devices can be lower, this in particular in the instance where DMSO is present. For example said concentration of the release-enhancing agent (excluding the DMSO content) may be in the range from about 1% to about 30% , or from about 1% to about 20%, or of about 2% to about 15%, or of about 5% to about 10%, e.g. about 5% or about 10%. All % in this paragraph are w/w relative to the total weight of the implantable device.

The concentration of the biocompatible, biodegradable polymer in the implantable devices of this invention may be in the range from about 10% to about 80%, or from about 10% to about 50%, or from about 10% to about 40%, or from about 20 to about 40%, e.g. about 20%, about 25%, about 30%, or about 40%. All % in this paragraph are w/w relative to the total weight of the implantable device.

TMC278 can be used in base-form or as pharmaceutically acceptable salt form, in particular as an acid addition salt form. Whenever mentioned herein, the term "TMC278" or "rilpivirine" refers to the base-from as well as to a pharmaceutically acceptable salt form. In one embodiment, TMC278 is used in base-form.

The devices in accordance with the present invention without the addition of the specific release-enhancing agents mentioned above do not, or insufficiently, release TMC278. The devices of the invnetion in particular is used at time intervals that are in the range of once a month to once every three months. Devices for administration in such time intervals preferably contain higher loads (or concentrations) of TMC278 as to keep the devices compact. It has been found that such TMC278 high-load devices can be made, but TMC278 is only released by the addition of the specific release-enhancing agents mentioned above.

The implantable devices of the invention result in a steady release of TMC278 from the device allowing effective blood plasma levels for a long time period. Release of TMC278 starts immediately after the device having been implanted, i.e. with limited or no delay. The implantable devices have the advantage that they can be removed from the body in case of adverse drug reactions. Devices without the release-enhancing agent have been found to not or inadequately release TMC278, which is assumed to be due to the hydrophobic nature of the implant material. It is assumed that because of the lipophilicity of TMC278, penetration of aqueous media in the implant material is hampered, in particular in the case of high loads ofTMC278. Only the specific release-enhancing agents mentioned above result in a good release profile of TMC278.

The implantable devices of the invention additionally show sufficient consistency and flexibility so that they can be manipulated, administered to, and, if desired, removed from the body. More than one device can be implanted, either at the same point in time or at different points in time. If multiple devices are implanted, these can be of smaller size. The number of devices that are implanted will not be unreasonable high, for example not more than 5, or not more than 2.

The implantable devices of the invention comprise a biocompatible, biodegradable polymer. Parameters of the polymer can be chosen to control the rate of degradation of the device. For example, lower initial molecular weights of the polymer and co-polymer can be used when the desire is for a faster degrading molecular weight. The monomer ratio in the co-polymer is another way to control the rate of degradation of a polymer. Polymer can be end-capped for added control of rate of degradation.

Biodegradable polymers readily break down into small segments when exposed to moist body tissue. The segments then either are absorbed by the body, or passed by the body. More particularly, the biodegraded segments do not elicit permanent chronic foreign body reaction, because they are absorbed by the body or passed from the body, such that no permanent trace or residual of the segment is retained by the body. Biodegradable polymers can also be referred to as bioabsorbable polymers, and both terms can be used interchangeably within the context of the present invention.

Suitable biocompatible, biodegradable polymers comprise aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, and blends thereof. For the purpose of this invention aliphatic polyesters include but are not limited to homopolymers and copolymers of lactide (which includes lactic acid, d-, 1- and meso lactide), glycolide (including glycolic acid), ε-caprolactone, p-dioxanone (1,4- dioxan-2-one), and trimethylene carbonate (1, 3-dioxan-2-one). In one embodiment, the biocompatible, biodegradable polymers are copolymers of lactide (which includes lactic acid, d-, 1- and meso lactide) and glycolide (including glycolic acid). In another embodiment, the biocompatible, biodegradable polymer is a copolymer of lactide and glycolide in a molar ratio of about 65% lactide to about 35% glycolide.

The implantable devices of the invention contain one or more specific release-enhancing agents. These agents are of the surfactant and/or emulsifier type. They are mixed with the biocompatible, biodegradable polymers. In one embodiment, the one or more specific release-enhancing agents are finely dispersed into the biocompatible, biodegradable polymer. The release-enhancing agent may also be dispersed into the biocompatible, biodegradable polymer as molecular dispersions, for example by melting the release-enhancing agent with the biocompatible, biodegradable polymer and further processing the thus-formed melt, e.g. by melt-extrusion.

The TMC278 active ingredient is similarly incorporated into the biocompatible, biodegradable polymers. In one embodiment, the TMC278 is finely dispersed into the biocompatible, biodegradable polymer. The TMC278 may be added to the biocompatible, biodegradable polymers or to a mixture of the biocompatible, biodegradable polymers and the one or more release-enhancing agents. If DMSO is used, the TMC278 may first be mixed with the DMSO and this mixture added to the polymer and the release-enhancing agent mixture. The DMSO may also be added to the polymer and the release-enhancing agent mixture after which the TMC278 is added. Preferably the polymer or polymers are molten while the TMC278 is added. Also here the formed mixture can be further processed such as by melt-extrusion.

One type of release-enhancing agents that can be added to the device is selected from the group of poloxamers, also known by the trade name Pluronic^{TM}(BASF). Poloxamers are nonionic triblock copolymers composed of a central hydrophobic chain of polyoxypropylene (polypropylene oxide) flanked by two hydrophilic chains of polyoxyethylene (polyethylene oxide), whith varying lengths of the polymer blocks. For the generic term "poloxamer", these copolymers are commonly named with the letter "P" (for poloxamer) followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content). Poloxamers are commercially available under the tradename Pluronic^{TM}. For the Pluronic tradename, coding of these copolymers starts with a letter to define its physical form at room temperature (L = liquid, P = paste, F = flake (solid)) followed by two or three digits. The first digit (two digits in a three-digit number) in the numerical designation, multiplied by 300, indicates the approximate molecular weight of the polyoxypropylene hydrophobe. The last digit, when multiplied by 10, indicates the approximate ethylene oxide content in the molecule (e.g., F127 = Pluronic^{TM} with a polyoxypropylene molecular weight of 3,600 g/mol and a 70% polyoxyethylene content). Pluronic™ F127 corresponds to poloxamer P407 (P407).

In one embodiment, the poloxamers have a polyoxypropylene molecular weight that is in the range of about 3,000 to about 4,800 g/mol and a polyoxyethylene content that is in the range of about 70% to about 80%. In one embodiment, the Pluronic™ (available from BASF) that is used is the F127 or the F 68 grade, and in particular is the F108 grade.

Another type of release-enhancing agents that can be added to the device is selected from the group ofpolysorbates. These are oily liquids derived from PEG-ylated sorbitan, which is a mixture of ingredients obtained from the dehydration of sorbitol) esterified with fatty acids. Examples include Polysorbate 20 (Tween™ 20 or polyoxyethylene (20) sorbitan monolaurate), Polysorbate 40 (Tween™ 40 or polyoxyethylene (20) sorbitan monopalmitate), Polysorbate 60 (Tween™ 60 or polyoxyethylene (20) sorbitan monostearate), and Polysorbate 80 (Tween™ 80 or polyoxyethylene (20) sorbitan monooleate). The number 20 following the polyoxyethylene part refers to the total number of oxyethylene -(CH₂CH₂O)- groups found in the molecule. The number following the polysorbate part is related to the type of fatty acid associated with the polyoxyethylene sorbitan part of the molecule. Monolaurate is indicated by 20, monopalmitate is indicated by 40, monostearate by 60 and monooleate by 80.

Another type of release-enhancing agents that can be added to the device is selected from a mixture of DMSO and one or more polymers selected from the group of polyvinyl-pyrrolidine polymers, also known as povidone (PVP). These are commercially available and have a molecular weight that is in the range of about 2,5 kD to about 2,500 kD. Examples are PVP K25 (BASF, MW=29,000), PVP K30 (BASF, MW=40,000), and PVP K90 (BASF, MW=360,000), available under the tradename Kolidon™. Of interest are PVPs having a molecular weight that is in the range of about 250 kD to about 500 kD; or of about 300 kD to about 400 kD. Of particular interest is PVP K90. Implants with only PVP as release-enhancing agent resulted in insufficient release of TMC278.

Further excipients can be added to the implant in minor quantity include biocompatible substances such as, e.g. surfactants, emulsifiers, hydrophilic polymers, or small molecules that are miscible with water. Suitable excipients include, but are not limited to polysorbates, sorbitan esters, mono and difatty acid esters, anionic surfactants, lipids, triglycerides, polyethylene glycols, hydrophilic polymers, such as poly(vinyl alcohol), and mixtures thereof Minor quantity in this context refers to a quantity of less than 10%, or less than 5%, or less than 2%, or less than 1%, any of these w/w, of such ingredients to the total weight of the implant.

In one embodiment the release-enhancing agents are combined with DMSO. For PVP addition of DMSO is a necessity in order to have acceptable release of TMC278 from the implant. The quantity of DMSO that is combined with release-enhancing agents may be in the range of about 2% to about 15%, or of about 3% to about 15%, or of about 3% to about 10% , or about 5% to about 10%, e.g. about 10%; each percentage mentioned in this paragraph being weight/weight relative to the total weight of the implantable device.

The implantable device of the invention is solid in form such that it may be easily be implanted and removed in case of an adverse event such as an allergic reaction to the TMC278. The shape of the dosage form is selected such that it allows convenient administration or removal. In one embodiment the device takes the form of a rod, i.e. an elongated cylinder with a small diameter, e.g. a diameter that is in the range of about 0.5 mm to about 6 mm, or of about 1 mm to about 5 mm, or of about 1.5 mm to about 4 mm, or of about 2 mm to about 3 mm. The length of the cylinder may vary, e.g. it can be in the range of about 1 cm to about 5 cm, or of about 2 cm to about 5 cm, or of about 2 cm to about 4 cm, or of about 2.5 cm to about 3.5 cm, e.g. about 3.5 cm, or about 3.0 cm, or about 2.5 cm. In another embodiment, the cylinder takes a coin-like (flat cilinder) shape. In that instance the height varies between about 1 mm and 10 mm, or between 2 mm and 5 mm, or 1.5 and 4 mm, while the diameter is in the range of about 10 mm to about 25 mm, or of about 10 mm to about 20 mm, or of about 15 mm to about 20 mm.

The volume of the implantable device also determines its shape. The volume of the device the device is equal or greater than 0.1 cc, or is equal or greater than 0.2 cc, or is equal or greater than 0.4 cc, or is equal or greater than 0.5 cc, or is equal or greater than 0.8 cc, or is equal or greater than 1 cc, or is equal or greater than 1.2 cc, or is equal or greater than 1.5 cc. In one embodiment the volume of the implantable device is about 1 cc. Too large volumes are not practicable, an upper limit may be 2 cc or 1.5 cc. As used herein cc means cubic centimeter.

In the event that the patient does not have any adverse effect, the device will remain until the polymer is completely degraded. The polymer degradation products and any remaining wetting agent or other excipient will be absorbed by the body without the need for subsequent removal once all of the drug is released.

The implantable device can be prepared by melt blending the biocompatible, biodegradable polymer, the wetting agent, the TMC278, and other excipients, if any, using conventional techniques, such as melt blending using an appropriate mixer and hot melt extrusion. The device material is then extruded through a die and cut into the desired length.

The administration of TMC278 as in the present invention may suffice to suppress HIV infection, but in a number of cases it may be recommendable to co-administer other HIV inhibitors. The latter preferably include HIV inhibitors of other classes, in particular those selected from NRTIs, PIs and fusion inhibitors. Co-administration may be oral or parenteral.

In certain instances, the treatment of HIV infection may be limited to only the administration of an implantable device in accordance with the invention i.e. as a monotherapy without co-administration of further HIV inhibitors. This option may be recommended, for example, where the viral load is relatively low, for example, where the viral load (represented as the number of copies of viral RNA in a specified volume of serum) is below about 200 copies / ml, in particular below about 100 copies / ml, more in particular below 50 copies / ml, specifically below the detection limit of the virus.

Alternatively, the invention can be used in the prevention against transmission of HIV similarly as described in WO 2006/106103. As noted, for the prevention against transmission the plasma levels of TMC278 should be kept above a minimum plasma level of 4 ng/ml, or 10 ng/ml, or 15 ng/ml, or 20 ng/ml, or 40 ng/ml. The blood plasma levels of TMC278 should preferably be kept above these minimum blood plasma levels because at lower levels the drug may no longer be effective thereby increasing the risk of transmission of HIV infection. Plasma levels of TMC278 may be kept at somewhat higher levels to have a safety margin and to avoid the development of mutated HIV, e.g. above a minimum plasma level of 93 ng/ml.

In a further aspect the implantable device can be employed together with an oral formulation (e.g. a tablet) of TMC278 or even with an oral formulation with a combination of HIV inhibitors. The oral formulation of TMC278 will immediately raise the plasma levels up to the minimum required level, and the implantable device can maintain the minimum required level for a sustained period of time. The device can be administered intermittently at a time interval that is in the range of two weeks to six months. However, if side effects are apparent the oral can be discontinued and the implantable can be immediately removed.

The implantable device of the invention is administered intermittently at a time interval of at least two weeks, or in particular at a time interval mentioned herein, meaning that the implantable device can be administered without any interjacent additional administrations of TMC278. Or in other words, implantable device of the invention can be administered at particular points in time separated from one another by a time period of at least two weeks, or in particular at a time interval as mentioned herein, during which no TMC278 can be administered. Such administration schedule is simple, requiring few administrations and therefore dramatically reduces the problem of "pill burden" faced with standard HIV medication. This in turn will improve the patient's compliance to the prescribed medication.

The implantable device of the invention can be administered (or implanted) at time intervals mentioned above. In one embodiment the time interval is in the range of two to three weeks, or three to four weeks. In another embodiment the time interval is in the range of one to two months, or two to three months, or three to four months, or four to six months. The time interval may be several weeks, e.g. 2, 3, 4, 5, or 6 weeks, or one or several months, e.g. 2, 3, 4, 5, or 6 months or even longer, e.g. 7, 8, 9, or 12 months.

As used herein the terms "treatment of HIV infection" or "suppression of HIV infection" relates to a situation of the treatment of a subject being infected with HIV. The term "subject" in particular relates to a human being.

Preferably, the implantable device is administered in a single administration, for example by one injection or implantation after a time interval of at least two weeks, e.g. by one injection or implant every two week or every month.

The dose ofTMC278 administered, which is the amount ofTMC278 in the implantable device of the invention, is selected such that the blood plasma concentration of TMC278 is kept during a prolonged period of time above a minimum blood plasma level. The term "minimum blood plasma level" in this context refers to the lowest efficacious blood plasma level, the latter being that blood plasma level of TMC278 that provides effective treatment of HIV, or in alternate wording, that blood plasma level of TMC278 that is effective in suppressing HIV. In particular, the blood plasma level of TMC278 is kept at a level above a minimum blood plasma level of about 10 ng/ml, or about 15 ng/ml, or about 20 ng/ml, or about 40 ng/ml. In a particular embodiment, the blood plasma level of TMC278 is kept above a level of about 93 ng/ml.

The plasma levels of TMC278 should be kept above these threshold blood plasma levels because at lower levels the drug may no longer be effective thereby increasing the risk of mutations. The dose of TMC278 administered also depends on the time interval at which it is administered. The dose will be higher where administration are less frequent.

The dose to be administered should be calculated on a basis of about 10 mg/day to about 200 mg/day, or about 20 mg/day to about 125 mg/day, e.g. about 25 mg/ day or about 100 mg/day, in particular 25 mg, or 50 mg, or 93 mg/day. These doses have to be multiplied by 7 for weekly doses and by 30 for monthly doses.

It has been found that the implantable devices of the invention result in blood plasma levels of TMC278 that are more or less stable, i.e. they fluctuate within limited margins and stay at about the same level during a long period of time, thereby approaching zero order release.

The TMC278 containing devices in accordance with this invention can be implanted subcutaneously by appropriate devices such as an injector needle of sufficient diameter or via a trocar, or by intruding into a small incision. The TMC278 implants can also be removed if necessary by a scalpel making a small incision in the skin and using a forceps or clamp to pull the device through the incision and suturing it shut.

In a further aspect, it was found that, although all of the release-enhancing agent are potentially sensitive to radical formation, and through this mechanism potentially degrade the TMC278, a gamma irradiation terminal sterilization method was found that did not result in TMC278 degradation (see example 7).

As used herein the term "about" in relation to a numerical value has its usual meaning. In certain embodiments, the term "about" can be left out and the numerical value itself should be applied. In other embodiments, the term "about" means the numerical value ± 10%, or ± 5%, or ± 2%, or ± 1%.

The following examples are meant to illustrate this invention, and should not be construed as a limitation as to its scope. The terms "device" and "formulation" are used interchangeably. The devices in accordance with the present invention are made of a formulation comprising the ingredients mentioned above.

### Example 1:

Six grams of poly(lactic co-glycolic acid) of monomer ratio 65/35 (inherent viscosity (IV) = 0.79 dl/g) was placed in a Brabender™ mixer with a volume of 30 cc. The mixer was heated to 100°C and the mixing blades were running at 60 rpm prior to the introduction of the polymer. After the polymer was introduced, 18 grams of TMC278 and 6 grams of Pluronic™ F108 (BASF) were fed into the mixture. Mixing continued at these pre-set conditions for an additional 5 minutes. The material was moved from the mixer, cooled at ambient conditions and subsequently fed in small portions into a DACA compounder. The barrel was pre-heated to 110°C and the screw speed was pre-set to 100 rpm. The extrudate strands were continuously collected, the diameter of the strands ranged from 1.5 - 2 mm. Strands were cut into samples containing 50 mg of TMC278, approximately 2.54 mm in length. Solid formulations were individually packaged in aluminum - lined packaging prior to sealing, the packages were purged and flushed with nitrogen overnight and sealed under nitrogen. Samples were terminally sterilized using gamma irradiation, with an exposure level of 15 kgy.

Various wetting agents were incorporated into the TMC278 and PLGA matrix using this melt processing method. These wetting agents included DMSO and DMSO with PVP. In these formulations the concentration of TMC278 was a constant 26% (w/w) of the total formulation and PLGA 65/35 varied from 73 to 74% of the total formulation. DMSO was added to formulations at 5 and 10% of total formulation, and Pluronic^{TM} F108 samples were prepared at 20% levels. All percentages mentioned in this example are (w/w) towards the total weight of the formulation.

### Example 2:

This example shows a study aimed at demonstrating that the administration of an implantable device of TMC278/F108/PLGA results in rapid uptake into blood plasma relative to the TMC278/PLGA. The study was performed in order to compare the plasma kinetics and the absolute bioavailability of TMC278 in the beagle dog after a single subcutaneous administration (SC) of 2 rods composed of 60% TMC278 / 20% PLGA 65/35 (IV = 0.79 dl/g) / 20% F108 relative to a single subcutaneous administration of 2 rods composed of 60% TMC278 / 40% PLGA 65/35 (IV = 0.79 dl/g). Six male beagle dogs (dog No. A1, A2, A3, B1, B2, B3), approximately 3 years old and weighing between 11 and 12 kg at the start of the experimental phase, were used in the present experiment. The dogs were dosed on the left flank. The area of implantation was first shaven and wiped down with ethanol and iodine solution. Animals were sedated with general anesthesia. The formulation was placed in a trocar with a 12 guage pointed needle. The needle was pushed under the skin and the formulation was released into the subcutaneous space. Two rods were placed in each dog for a total TMC278 dose of 8 - 9 mg/kg. The A group of beagles received the TMC278/PLGA formulation and the B group received the TMC278/F108/PLGA system.

Blood samples were taken from a jugular vein from the dog at specified time points after dose administration. After sampling, the blood samples were immediately placed on melting ice and protected from light. Blood samples were centrifuged at approximately 1900X g for 10 minutes at 5°C to allow plasma separation. Immediately after separation, plasma samples were protected from light, placed on melting ice and stored at ≤ -18°C.

The concentration of TMC278 in dog plasma was determined by a qualified research LC-MS/MS method after solid phase extraction (SPE). Plasma concentrations of TMC278 were determined after proper sample clean up. The sample (0.1 ml aliquots of plasma) was extracted using a solid phase extraction method (Bond Elut Certify solid phase columns, 130 mg, SPE, Varian). The SPE column was conditioned with a 3 ml methanol, 3 ml water, and 1 ml acetic acid (1 M). After addition of 3 ml acetic acid to 0.1 ml aliquots of plasma the samples were extracted on the column followed by washing the column with 1 ml water, 1 ml acetic acid (1 M), and 3 ml methanol. The column was eluted with 3 ml methanol / NH₄OH 25% (98:2 v/v). The extract was evaporated to dryness and reconstituted to 150 µl of ammonium formate, 0.01M (adjusted to pH 4 with formic acid / methanol (40:60)(v/v). The flow-rate to the mass spectrometer was about 100 µl / min after splitting. LC - MS/MS analysis was carried out on an API-3000 system (Applied Biosystems) which was coupled to an HPLC system.

The results of this experiment are summarized in Table 1. Results indicated a delay time prior to the detection of TMC278 in plasma for formulations in which the F108 was absent. The delay ranged from 7 - 21 days. The delay was followed by sustained plasma levels of TMC278 for the remainder of the time period of the experiment. In contrast, formulations with F108 demonstrated a more rapid absorption into the plasma.

**Table 1: Plasma concentrations in ng/ml of TMC278 in dogs**

| Time (hours) | Beagle A1 | Beagle A2 | Beagle A3 | Beagle B11 | Beagle B12 | Beagle B13 |
|---|---|---|---|---|---|---|
| 0 | <0.5 | <0.5 | <0.5 | <0.5 | <0.5 | <0.5 |
| 6 | <0.5 | <0.5 | <0.5 | <0.5 | <0.5 | <0.5 |
| 24 | <0.5 | <0.5 | <0.5 | 1.03 | 1.07 | <0.5 |
| 48 | <0.5 | <0.5 | <0.5 | 1.92 | 1.53 | <0.5 |
| 72 | <0.5 | <0.5 | <0.5 | 3.13 | 3.35 | <0.5 |
| 168 | <0.5 | <0.5 | 0.612 | 5.34 | 6.52 | 1.78 |
| 216 | <0.5 | <0.5 | 0.884 | 8.86 | 7.06 | 3.83 |
| 264 | <0.5 | <0.5 | 2.01 | 7.84 | 6.85 | 4.62 |
| 336 | <0.5 | 0.807 | 6.86 | 5.66 | 6.27 | 5.17 |
| 504 | 7.73 | 3.52 | 11.8 | 4.84 | 3.64 | 3.10 |
| 672 | 7.00 | 3.67 | 10.8 | 1.96 | 3.00 | 2.48 |
| 840 | 4.32 | 2.69 | 5.41 | 1.66 | 2.61 | 1.89 |
| 1032 | 6.08 | 1.45 | 3.38 | 1.35 | 1.74 | 1.35 |
| 1176 | 6.38 | 1.09 | 3.43 | 1.23 | 1.52 | 1.47 |
| 1344 | 4.51 | 0.832 | 2.70 | 1.23 | 1.75 | 1.26 |
| 1560 | 3.16 | 0.767 | 2.06 | 1.14 | 2.47 | 1.19 |
| 1680 | 3.09 | 0.580 | 2.00 | 0.989 | 1.61 | <0.5 |
| 1848 | 2.12 | 0.733 | 2.09 | 1.20 | 1.49 | 1.16 |
| 2016 | 2.35 | 0.999 | 6.64 | 2.32 | 4.12 | 2.34 |

### Example 3:

This example tests different formulations for their effect on rapid uptake into blood plasma after implantation. The study was performed in order to compare the plasma kinetics and the absolute bioavailability of TMC278 in Sprague-Dawley rats after a single subcutaneous administration (SC) of 1 rod composed either of 1) 60% TMC278 / 40% PLGA 50/50 or 2)60% TMC278 / 20% F108/ 20% PLGA 50/50 or 3)60% TMC278 / 10% DMSO / 30% PLGA 50/50 or 4)60% TMC278 / 10% DMSO / 5% PVP / 25% PLGA 50/50.

A device composed of drug and polymer and the drug / polymer device containing the F108 were prepared as described in a previous example. A device containing DMSO (dimethylsulfoxide) was prepared by initially feeding the 9 grams of PLGA 50/50 (IV = 0.79 dl/g) into the pre-heated (120°C) Brabender mixing bowl. After the polymer was fed into the mixer, eighteen grams of TMC278 with three grams of DMSO pre-mixed in it was added in powdered form to the mixer. Mixing continued at stated conditions for another 5 minutes. The mixture was then removed from the mixer, cooled to ambient conditions, and extruded using a Daca compounder. The extrudate was in rod shape with diameter 1 - 2 mm.

The device containing the excipient combination of DMSO and PVP (poly(vinylpyrrolidone)) was prepared in a similar fashion to the previously described devices. A 7.5 gram sample of PLGA 50/50 was placed into Brabender mixer bowl that was pre-heated to 100°C. A 1.5 gram sample of PVP was placed in the pre-heated bowl with the PLGA. Three grams of DMSO were added to the polymer mixture. The three components were mixed at 60 rpm for 5 minutes, when they reached a consistent formulation. Eighteen grams of TMC278 was added in powdered form and mixing continued for another 5 minutes. Mixture was removed from Brabender, cooled and extruded into strands of 1 - 2 mm using the Daca as described in previous experiment.

Eighty female Sprague-Dawley rats weighing 250 - 350 grams were used in the present study. Animals were initially anesthetized using inhalation anesthesia (Isoflurane at 5.0%). After induction of anesthesia, the surgical site of the animal from the dorsal cervical area to the dorsal lumbar area was clipped free of hair using an electric animal clipper. The area around the site of surgery was scrubbed with chlorhexidine diacetate, rinsed with alcohol, dried, and painted with an aqueous iodophor solution of 1% available iodine. An incision of approximate length 1 cm, was made on the dorsum of the thoracic region, about 2 cm caudal to the palpated inferior edge of the scapula. The skin was separated from the underlying connective tissue to make a small pocket. The rod was inserted through the incision into the subcutaneous space and implanted in place that is about 1 - 2 cm caudal to the incision. The skin incision was closed with 2 - 3 wound clips. Mass of implants were approximately 16 - 17 mg, mass of TMC278 in each implant was 9 - 10 mg to deliver a dose of approximately 20 mg/kg.

Rats were euthanized at designated intervals via inhalation of carbon dioxide. Subsequently, blood samples were collected via cardiac puncture from all rats at each time point. Samples were immediately placed on ice, protected from light, and centrifuged to extract the plasma within an hour of euthanasia. The TMC278 content in plasma was measured using the same method as that described in previous example for the dog plasma samples.

The results of the analysis of the plasma samples are summarized in Table 2. From these data it is apparent that detectable levels of MC278 were not observed until 4 weeks after implantation for samples composed of the drug and PLGA. However, each of the formulations containing excipients demonstrated detectable levels of TMC278 within 1 day. The highest plasma concentrations of TMC278 were observed when F108 was used. Formulations with DMSO but no PVP were associated with the lowest plasma levels. The addition of PVP to the DMSO-containing formulations dramatically increased the TMC278 plasma levels.

**Table 2: Plasma concentration of TMC278 in rats**

| Time (Days) | TMC278/ PLGA 50/50 | TMC278/F108/ PLGA 50/50 | TMC278/DMSO/ PLGA 50/50 | TMC278/DMSO/ PVP/PLGA 50/50 |
|---|---|---|---|---|
| 1 | BL | 1.77 | 1.73 | 2.07 |
| 1 | BL | 7.86 | 1.92 | 2.47 |
| 1 | BL | 3.61 | 1.65 | 1.42 |
| 1 | BL | 4.32 | BL | 1.24 |
| 7 | BL | 3.87 | BL | 1.60 |
| 7 | BL | 7.02 | BL | BL |
| 7 | BL | 7.28 | BL | 2.50 |
| 7 | BL | 16.3 | 1.49 | 2.39 |
| 14 | BL | 3.4 | BL | 3.36 |
| 14 | BL | 1.94 | BL | BL |
| 14 | BL | 2.85 | BL | 1.39 |
| 14 | BL | 1.23 | BL | 1.08 |
| 21 | BL | 1.59 | 2.28 | 1.39 |
| 21 | BL | 3.03 | 1.35 | 1.66 |
| 21 | BL | 1.34 | BL | 1.55 |
| 21 | BL | 1.9 | 2.19 | 1.26 |
| 28 | 3.49 | 1.99 | 1.43 | 2.35 |
| 28 | 2.15 | 1.19 | BL | 1.25 |
| 28 | 2.11 | 1.39 | BL | 1.28 |
| 28 | BL | 2.54 | BL | 1.36 |

| | | | | |
|---|---|---|---|---|
| BL means below level of quantification (0.5 ng/ml) | | | | |

### Example 4

Formulations containing TMC278 and PLGA 50/50 with and without DMSO, and an additional formulation containing DMSO with PVP were prepared as described in the previous example. Samples were incubated in PBS for 4 weeks, rinsed and dried. Following drying the surfaces of and cross-sections of the samples were analyzed using scanning electron microscopy. After 4 weeks of in vitro incubation significant degradation occurs to the devices. Surprisingly, examination of the 60/40 TMC278/PLGA samples demonstrated large pores and voids developed around the outer circumference of the rod as if the device was degrading form the surface and into the bulk of the matrix. The addition of a minimum of 10% (w/w) DMSO results in the pores, channels, voids developing across the entire cross-section of the device during incubation. At DMSO concentrations below 10% the developing pores concentrate around the outer circumference of the device. Increasing the concentration to 10% (w/w) DMSO increases the "wettability" of the matrix sufficiently for water to penetrate the bulk of the matrix. The addition of PVP to a device that already contains 10% DMSO results in even larger voids and pores (10 - 100 micron in diameter) developing across the bulk giving the appearance of a foam. This behavior suggests that when no excipient is used the aqueous surrounding fluid required to penetrate the device and extract the drug is concentrated at the surface of the device due to the significant hydrophobic nature of the polymer and drug. The addition, of an excipient increases the wettability of the bulk of the device allowing aqueous fluid to penetrate the entire device and provide for a means of drug diffusion. If absorption of the aqueous fluid is prevented by the hydrophobicity of the device then the only path by which the drug can diffuse out of the matrix is after the polymer has degraded sufficiently to allow aqueous fluid to penetrate into the interior of the device. This can account for the long delay between implantation and when detectable plasma levels are observed for devices without excipients.

### Example 5

In addition to increasing the water uptake into the bulk of the polymer, excipients can be used to decrease the crystallinity of the TMC278 and thereby lower the energy necessary to solubilize the drug. The TMC278 is highly soluble in DMSO, and therefore it can be used to "recrystallize" TMC278 into either an amorphous morphology or one with reduced crystallinity. The recrystallized TMC278 was prepared by dissolving 10 grams of TMC278 in 800 ml of DMSO under gentle stirring for 2 hours. One hundred milliliters of the solution was subsequently poured into a flat bottomed aluminum mold. Solution was lyophilized using a Dora-Stop MTS system. Lyophilized TMC278 was collected. Two grams of PLGA 50/50 were fed into DACA compounder that was pre-set to 120°C with screws rotating at 100 rpm. After the polymer was fed into the DACA and melted, two grams of lyophilized TMC278 were fed into the compounder and mixed at the set conditions for an additional 5 minutes. The extruded strands were collected, cooled in ambient conditions and placed in plastic bags and stored in a nitrogen box for analysis.

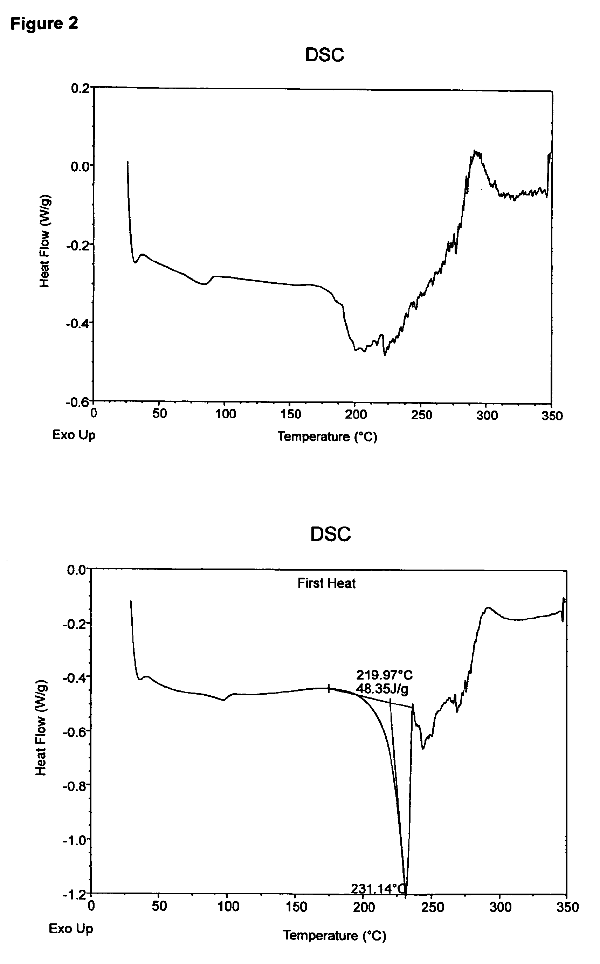

Differential scanning calorimetery was used to test the difference in crystallinity of TMC278 after melt processing with the PLGA. The test PLGA 50/50 samples contained 50% (w/w) TMC278 that had been recrystallized from DMSO and 10% (w/w) residual DMSO. Control PLGA 50/50 samples contained 60% (w/w)TMC278 and 10% (w/w) DMSO that had been blended into the PLGA as described in previous examples. The first heat thermograms of the two samples (Figure 2) are clearly different. The melting point of TMC278 when DMSO is blended into the matrix is 231°C and clearly defined. In contrast, no clearly defined melting point for TMC278 is observed when the re-crystallized TMC278 is dispersed in the PLGA.

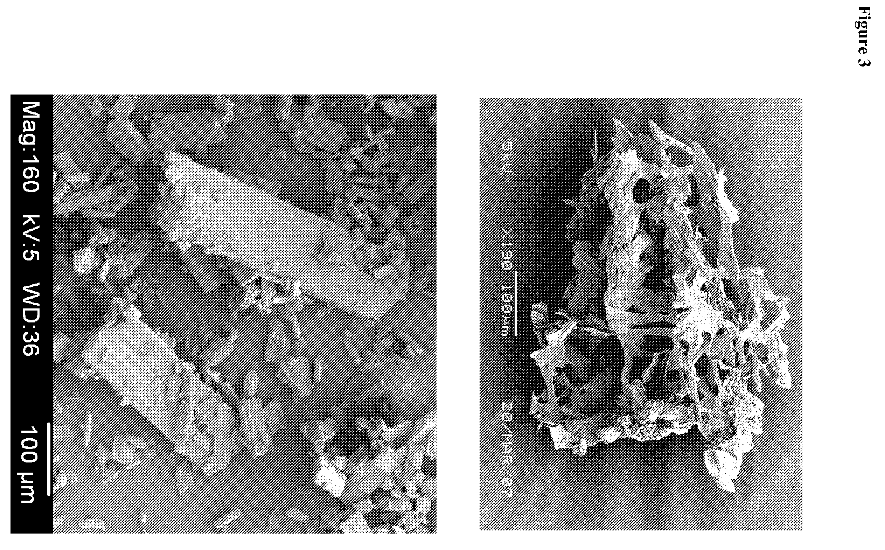

The lowered crystallinity of the TMC278 when re-crystallized from DMSO is also reflected in the change of the appearance of the TMC278 crystals following the recrystallization procedure. The morphology of the TMC278 particles in the drug powder appear compact and needle-shaped, but after the recrystallization process the particles are highly porous (Figure 3). This porous morphology corresponds to a dramatic increase in surface area and therefore an increase in the amount of drug that is exposed to dissolution media and therefore a higher solubility. To test this effect the solubility of the recrystallized TMC278 was tested and compared with that of unrecrystallized TMC278 and found to be more than 250x more soluble.

### Example 6: Study with various potential release-enhancing agents

### Poly(monooleoylglyceride co-succinate co-poly(ethylene glycol) (MGSA co-PEG).

12 g of poly(lactide co-glycolide) (50/50) was fed into a 30 cc Brabender mixer that was pre-heated to 70°C and with twin screw blades pre-set to 60 rpm. Subsequently 9 g of MGSA co-PEG was added followed by 9 g of TMC278. This polymer surfactant was a 1:1 ratio of poly(monostearoyl glycerol co-succinate) and poly(ethylene glycol). The number average of the polyethylene glycol used to prepare the polymer was 2000 daltons. Once all components were added, the temperature of the mixing bowl was raised to 100°C and the content of the bowl was allowed to mix for an additional 8 minutes. The mixed samples were then taken out of the mixer, cooled in ambient conditions and fed as small pieces into a Daca compounder to extrudate strands for testing. The temperature of the Daca was pre-set to 65°C and the screw speed was set to 100 rpm. The extrudate was continuously collected as strands of approximately 2 mm in diameter.

Samples of the extrudate were assayed for TMC278 content. Five samples, 25 mg in mass, were cut from the extrudate and dissolved in DMSO. The DMSO completely dissolved the entire extrudate. The solution was analyzed using a Perkin Elmer Series 200 HPLC fitted with a Discovery C18 column of dimensions 3.0 mm x 150 mm x 5 micron (s/n 105153-01). The mobile phase of the isocratic method consisted of 55% water and 40% acetonitrile, the acetonitrile also consisted of 0.1% formic acid and 10 mM of ammonium formate. The mobile phase was pumped at 0.4 ml/min, column was heated to 30°C and detector was set at 288 nm. The average content of the TMC278 in the five samples was 30% (w/w) with a standard deviation of 3%.

### Polysorbate 80

Nine grams of Polysorbate 80 were pre-mixed with 9 grams of TMC278 to form a paste prior to be being compounded with the polymer. Twelve grams of poly(lactide co-glycolide) 50/50 was fed into a Brabender mixer that was pre-heated to 70°C and with screws pre-set to 60 rpm. The paste was added to the warm polymer, the temperature was raised to 100°C and contents mixed for an additional 8 minutes. The mixture was scraped out of the mixer, cooled at ambient and extruded into strands as described in previous example. Samples of the extrudate were assayed for TMC278 content. Five samples, 25 mg in mass, were cut from the extrudate and dissolved in DMSO. The DMSO completely dissolved the entire extrudate. The solution was analyzed using HPLC as described above. The average content of the TMC278 in the five samples was 31 % (w/w) with a standard deviation of 0.9%.

### Vitamin E-TPGS

Twelve grams of PLGA 50/50 were fed into the Brabender mixer that was pre-heated to 70°C and with screw speed pre-set to 60 rpm. Subsequently 9 grams of Vitamin E TPGS was added to the polymer followed by the addition of 9 grams of TMC278. After all components were added to the bowl the temperature of the mixing bowl was raised to 100°C and the contents were allowed to mix for an additional 5 minutes. The mixture was scraped out of the mixer, cooled at ambient and extruded into strands as described in first example. Samples of the extrudate were assayed for TMC278 content as described above. Five samples, 25 mg in mass, were cut from the extrudate and dissolved in DMSO. The DMSO completely dissolved the entire extrudate. The average content of the TMC278 in the five samples was 27% (w/w) with a standard deviation of 1.4%.

### Dimyristoylphophatidylcholine (DMPC)

12 g of poly(lactide co-glycolide) 50/50 were fed into a Brabender twin screw mixer that was pre-heated to 70°C and 60 rpm. Subsequently, 9 g of DMPC were added with 9 g of TMC278 to the mixing polymer. The temperature of the mixing bowl was raised to 100°C and the content was allowed to mix for an additional 5 minutes. The mixture was scraped out of the mixer, cooled at ambient and extruded into strands as described in first example. Samples of the extrudate were assayed for TMC278 content as described above. The average content of the TMC278 in the five samples was 19% (w/w) with a standard deviation of 1.02%.

### Caprolactone co-trimethylencarbonate co-Poly(ethylene glycol) (Cap-TMC-PEG) (composition description can be found in US2006/0034797).

12 g of poly(lactide co-glycolide) 50/50 was fed into a Brabender twin screw mixer that was pre-heated to 70°C and 60 rpm. Subsequently, 9 g of Cap-TMC-PEG followed by 9 g of TMC278 were added to the warmed and mixing polymer. The composition of this batch of the polymer surfactant was 1 mole caprolacton, 1 mole trimethylene carbonate and 0.15 mole poly(ethylene glycol). The number average of poly(ethylene glycol) used in the synthesis of the polymer was 750. The molecular weight of the polymer surfactant, Cap-TMC-PEG was 5800 daltons. The temperature of the mixing bowl was raised to 100°C and the content was allowed to mix for an additional 5 minutes. The mixed samples were taken out of the mixer, cooled in ambient conditions and extruded into strands as described in the first example. Samples of the extrudate were assayed for TMC278 content as described above. The average content of the TMC278 in the five samples was 27% (w/w) with a standard deviation of 0.81%.

### F108

6 g of PLGA 50/50 was placed in mixing bowl of a Brabender mixer that was pre-set to 100°C and 60 rpm. Subsequently 18 grams of TMC278 was added followed by 6 grams of F108 polymer. Mixing was continued for 5 minutes after all components were added. The sample was removed from mixer, cooled to ambient temperature and fed into a Daca compounder that was preset to 80°C and 100 rpm. Samples of the extrudate were assayed for TMC278 content as described above, The average content of the TMC278 in the five samples was 55% (w/w) with a standard deviation of 5.02%.

### Control Samples composed of TMC278 and PLGA 50/50

12 g of PLGA 50/50 were placed in mixing bowl of a Brabender mixer that was pre-set to 100°C and 60 rpm. Subsequently 18 g of TMC278 was added. Mixing continued for 5 minutes after all components were added. The sample was removed from the mixer, cooled to ambient temperature and fed into a Daca compounder that was preset to 80°C and 120 rpm. Samples of the extrudate were assayed for TMC278 content as described above. The average content of the TMC278 in the five samples was 55% (w/w) with a standard deviation of 1.6%.

The various polymer implants described above were implanted into the scapular region of a group of 5 Sprague Dawley male rats (250 - 350 grams) at a respective dose of 80 mg/kg. Blood samples from the tail vein of each rat in the groups were taken at 3 hours, 24 hours, 48 hours, 7 days, 14 days, 21 days and 28 days. After the blood sample was taken it was centrifuged to separate out the plasma. The TMC278 was extracted from the plasma and analyzed for content. The results are summarized in Table 3.

**Table 3: Plasma levels of TMC278 from polymer implants containing surfactants, dosed at 80 mg/kg in a rat model**

| Time (Days) | MGSA co-PEG | Polysorbate 80 | DMPC | CAP-TMC-PEG | F108 | Vitamin E TPGS | Control |
|---|---|---|---|---|---|---|---|
| 0.125 | 4.44 | 15.82 | 7.45 | 9.70 | 4.20 | 16.07 | 1.43 |
| 1 | 1.34 | 3.50 | 3.84 | 3.11 | 0.68 | 0.75 | 0.75 |
| 3 | 0.46 | 2.29 | 1.11 | 0.72 | 0.5 | 1.0 | 0.30 |
| 7 | 0.53 | 1.39 | 1.88 | 0.1 | n/a | 0.52 | n/a |
| 8 | n/a | n/a | n/a | n/a | 3.59 | n/a | 0.26 |
| 14 | 0.63 | 1.83 | 1.55 | 0.38 | 3.56 | 0.48 | 0.25 |
| 21 | 0.45 | 3.37 | 1.66 | 0.52 | 3.25 | 0.50 | 0.10 |
| 28 | 0.49 | 3.37 | 1.68 | 0.66 | 3.60 | 0.54 | 0.12 |

| | | | | | | | |
|---|---|---|---|---|---|---|---|
| n/a = sample was not taken at that timepoint | | | | | | | |

Results of the experiments comparing the various surfactants on the plasma level of TMC278 indicated that all surfactants demonstrated a higher initial TMC278 plasma level relative to the control samples without a surfactant. In fact, the increased dose used in this experiment resulted in eliminating the lag time even in the control samples, though after peaking at the 3 hour time point a steady decrease in plasma levels was observed for the duration of the experiment. Polysorbate 80 and Vitamin E TPGS were associated with the highest initial plasma levels of TMC28, both about 16 ng/ml, however only the Polysorbate 80 and the F108 were able to maintain the highest plasma levels of TMC278 over the 28 days.

The F108 samples in addition to the controls were tested in one study previous to the study testing the other enhancers since there was a limit to the number of animals that could be tested simultaneously. The composition of the samples in the earlier study were 60% TMC278 and 40% (w/w) PLGA 50/50 in the case of the control and 60% (w/w) TMC278, 20% (w/w) F108, and 20% PLGA 50/50. In contrast, the other enhancer samples were prepared with a lower concentration (as noted below) of TMC278. This was done since it was not possible to process high loadings of TMC278 for some of the other excipients. Therefore the concentration of TMC278 for the enhancer samples included in the same study was reduced to 20 - 30 % (w/w). The concentration of enhancer was increased to 30% w/w in order to provide the best possible chance for the surfactant to affect the solubility. Interestingly, even at lower concentration relative to the other enhancers, the F108 demonstrated amongst the highest performers for the long term higher levels of TMC278.

### Example 7: Sterilization Study

For those samples that were irradiated under a nitrogen atmosphere, the following procedure was followed. Polymer implants were prepared containing 60% TMC278 and 40% PLGA 50/50 as described above. Samples were placed in Nalgene™ cuvettes inside the antechamber of a nitrogen glovebox. An automatic vacuum cycle was executed consisting of three 8 minute vacuum purges each followed by nitrogen refilling. The samples were transferred to the main chamber and allowed to equilibrate overnight. The cuvettes were placed in foil pouches and the pouches were sealed before removal from the nitrogen glovebox. Samples were removed from glovebox and irradiated as directed.

Those samples that were irradiated under ambient conditions were placed in vials and aluminum pouches, which were heat sealed under ambient conditions and were stored at 0°C until they reached that temperature. The samples that were processed at 0°C were stored at 0°C. Subsequently, the samples were removed from freezer environment and immediately placed in the irradiator. Following irradiation the samples were assayed for TMC278 content. The results are summarized in Table 4.

**Table 4: Testing of Effect of Gamma Irradiation Process Parameters on Recovery of TMC278 from Polymer Specimens**

| Exposure level (kgy) | Process environment | Process Temperature | TMC278 recovery |
|---|---|---|---|
| 25 | Nitrogen | 0°C | 90% |
| 25 | Nitrogen | 0°C | 89% |
| 25 | Nitrogen | Room Temp | 85% |
| 25 | Nitrogen | Room Temp | 78% |
| 25 | Ambient | Room Temp | 82% |
| 25 | Ambient | Room Temp | 77% |
| 15 | Nitrogen | Room Temp | 94% |
| 15 | Nitrogen | Room Temp | 105% |
| control | N/A | N/A | 100% |
| control | N/A | N/A | 100% |

From the data it can be seen that exposure level of irradiation is the most significant factor in achieving complete recovery of TMC278 from irradiated samples. Only 81 % of the TMC278 was recovered from samples that were irradiated at 25 kgy (under nitrogen) relative to 100% average recover of samples irradiated at 15 kgy (under nitrogen). For the two sets of samples that were irradiated at 25 kgy at ambient temperature there was only a slight increase in recovery from samples packaged under nitrogen versus samples packaged under ambient conditions. At 25 kgy, reduced temperature was important in achieving complete recovery of TMC278, however, at 15 kgy, since 100% was already achieved without reduced termperature, obviously, reduced temperature is not required. In addition, the samples that were exposed to 15 kgy irradiation under nitrogen environment were analyzed for impurities stemming from TMC278 degradation. A DE/AD MS 07 TSQ Quantum mass spectrometer was used to detect impurities and results indicated no new impurities formed by the irradiation.

Samples containing TMC278 and the various enhancers were irradiated in preparation for animal testing. The samples were irradiated at 15 kgy in a nitrogen environment and at ambient temperature as described above. Three samples of each type were tested at these conditions and compared against an average of three controls (non-irradiated) per sample type. The results are summarized in Table 5. There is virtually no difference between the irradiated and non-irradiated in the batches containing the F108, Vitamin E TPGS, Cap-TMC-PEG, and MGSA co-PEG. There is some variability between gamma and non-gamma irradiated batches in the case of those devices containing either DMPC or Polysorbate 80, however, since the error in the measuring process is about 5% it is not a dramatic difference.

**Table 5: Percentage by weight of TMC278 in irradiated samples versus non-irradiated samples that contained surfactants**

| | Average TMC278 content - Gamma (standard deviation from the mean) | Average TMC278 content - non-Gamma (standard deviation from the mean) |
|---|---|---|
| MGSA co-PEG | 29.6% (1.54) | 26.7% (0.89) |
| Polysorbate 80 | 35.3 (1.48) | 29.0 (0.28) |
| DMPC | 23.3 (8.81) | 18.6(0.32) |
| Cap-TMC-PEG | 26.0 (0.22) | 25.7 (0.46) |
| Vitamin E-TPGS | 26.6 (0.35) | 25.5 (0.7) |
| F108 | 61 (1.13) | 59.8 (0.64) |

## Claims

1. An implantable one piece device comprising a biocompatible, biodegradable polymer selected from homopolymers and copolymers of lactide, glycolide, ε-caprolactone, p-dioxanone (1,4- dioxan-2-one), and trimethylene carbonate (1, 3-dioxan-2-one) mixed with rilpivirine in base-form or as pharmaceutically acceptable salt form and with one or more release-enhancing agents selected from the group consisting of poloxamers, polysorbates, and a combination of dimethyl sulfoxide (DMSO) and poly(vinyl pyrrolidone)(PVP), wherein the device contains from 25% by weight to 70 % by weight of rilpivirine in base form or as pharmaceutically acceptable salt from.

2. The device of claim 1, wherein the device weighs more than 100 mg.

3. The device of claim 1, wherein the device weighs more than 500 mg.

4. The implantable device of any of claims 1 - 3, shaped as a cylinder.

5. The implantable device of claim 4, having a diameter that is in the range of 0.5 mm to 4 mm, and a length that is in the range of 1.0 cm to 4 cm.

6. The implantable device of claim 4, having a diameter that is in the range of 1.0 mm to 3.0 mm, and a length that is in the range of 1.5 cm to 3.5 cm.

7. The device of any of claims 1 - 6, wherein the device contains from 25% by weight to 60% by weight, or from 40% by weight to 60% by weight, or from 50% by weight to 60% by weight, of rilpivirine in base-form or as pharmaceutically acceptable salt form.

8. The device of any of claims 1 - 7, wherein the biocompatible, biodegradable polymer is selected from copolymers of lactide and glycolide.

9. The device of claim 8, wherein the biocompatible, biodegradable polymer is a copolymer of lactide and glycolide in a molar ratio of 50% to 65% lactide to 35% to 50% glycolide.

10. The device of any of claims 1 - 9, wherein the release-enhancing agent is a poloxamer.

11. The device of claim 10, wherein the release-enhancing agent is poloxamer 338.

12. The device of any of claims 1 - 11, wherein the device contains from 1% to 40% w/w relative to the total weight of the implantable device, or from 10% to 30% w/w relative to the total weight of the implantable device, or of 15% to 25% w/w relative to the total weight of the implantable device, of said release-enhancing agent.

13. The device of any of claims 1 - 12, wherein the device contains from 10% to 80% w/w relative to the total weight of the implantable device, or from 10% to 30% w/w relative to the total weight of the implantable device, or from 15 to 25% w/w relative to the total weight of the implantable device, e.g. 20% w/w relative to the total weight of the implantable device, of said biocompatible, biodegradable polymer.

14. The device of any of claims 1 - 13, wherein the device contains from 15 % to 25 % w/w relative to the total weight of the implantable device of biocompatible, biodegradable polymer.

15. The device of any of claims 1 - 14, wherein when present, the amount of DMSO is in the range of 3% to 10% w/w relative to the total weight of the implantable device.

16. The device of any of claims 1 - 15, for use in the treatment of HIV infection when administered intermittently at a time interval from 2 weeks to 3 months.

17. The device of any of claims 1 - 15, for use in the prevention against transmission of HIV when administered intermittently at a time interval from 2 weeks to 3 months.

18. The device of any of claims 1 - 16 wherein rilpivirine is used in base-form.

## Patentansprüche

1. Implantierbare einstückige Vorrichtung, umfassend ein biologisch kompatibles, biologisch abbaubares Polymer, ausgewählt aus Homopolymeren und Copolymeren von Lactid, Glycolid, ε-Caprolacton, p-Dioxanon (1,4-Dioxan-2-on) und Trimethylencarbonat (1,3-Dioxan-2-on) in Abmischung mit Rilpivirin in Basenform oder als pharmazeutisch annehmbare Salzform und mit einem oder mehreren freigabefördernden Agenzien, ausgewählt aus der Gruppe bestehend aus Poloxameren, Polysorbaten und einer Kombination aus Dimethylsulfoxid (DMSO) und Poly(vinylpyrrolidon)(PVP), wobei die Vorrichtung 25 Gew.-% bis 70 Gew.-% Rilpivirin in Basenform oder als pharmazeutisch annehmbare Salzform enthält.

2. Vorrichtung nach Anspruch 1, wobei die Vorrichtung mehr als 100 mg wiegt.

3. Vorrichtung nach Anspruch 1, wobei die Vorrichtung mehr als 500 mg wiegt.

4. Implantierbare Vorrichtung nach einem der Ansprüche 1-3 in Form eines Zylinders.

5. Implantierbare Vorrichtung nach Anspruch 4 mit einem Durchmesser im Bereich von 0,5 mm bis 4 mm und einer Länge im Bereich von 1,0 cm bis 4 cm.

6. Implantierbare Vorrichtung nach Anspruch 4 mit einem Durchmesser im Bereich von 1,0 mm bis 3,0 mm und einer Länge im Bereich von 1,5 cm bis 3,5 cm.

7. Vorrichtung nach einem der Ansprüche 1-6, wobei die Vorrichtung 25 Gew.-% bis 60 Gew.-%, oder 40 Gew.-% bis 60 Gew.-%, oder 50 Gew.-% bis 60 Gew.-% Rilpivirin in Basenform oder als pharmazeutisch annehmbare Salzform enthält.

8. Vorrichtung nach einem der Ansprüche 1-7, wobei das biologisch kompatible, biologisch abbaubare Polymer aus Copolymeren von Lactid und Glycolid ausgewählt ist.

9. Vorrichtung nach Anspruch 8, wobei es sich bei dem biologisch kompatiblen, biologisch abbaubaren Polymer um ein Copolymer von Lactid und Glycolid in einem Molverhältnis von 50% bis 65% Lactid zu 35% bis 50% Glycolid handelt.

10. Vorrichtung nach einem der Ansprüche 1-9, wobei es sich bei dem freigabefördernden Agens um ein Poloxamer handelt.

11. Vorrichtung nach Anspruch 10, wobei es sich bei dem freigabefördernden Agens um Poloxamer 338 handelt.

12. Vorrichtung nach einem der Ansprüche 1-11, wobei die Vorrichtung 1% bis 40% (w/w) in Bezug auf das Gesamtgewicht der implantierbaren Vorrichtung, oder 10% bis 30% (w/w) in Bezug auf das Gesamtgewicht der implantierbaren Vorrichtung, oder 15% bis 25% (w/w) in Bezug auf das Gesamtgewicht der implantierbaren Vorrichtung des freigabefördernden Agens enthält.

13. Vorrichtung nach einem der Ansprüche 1-12, wobei die Vorrichtung 10% bis 80% (w/w) in Bezug auf das Gesamtgewicht der implantierbaren Vorrichtung, oder 10% bis 30% (w/w) in Bezug auf das Gesamtgewicht der implantierbaren Vorrichtung, oder 15% bis 25% (w/w) in Bezug auf das Gesamtgewicht der implantierbaren Vorrichtung, z.B. 20% (w/w) in Bezug auf das Gesamtgewicht der implantierbaren Vorrichtung, des biologisch kompatiblen, biologisch abbaubaren Polymers enthält.

14. Vorrichtung nach einem der Ansprüche 1-13, wobei die Vorrichtung 15% bis 25% (w/w) in Bezug auf das Gesamtgewicht der implantierbaren Vorrichtung an biologisch kompatiblem, biologisch abbaubarem Polymer enthält.

15. Vorrichtung nach einem der Ansprüche 1-14, wobei die Menge an DMSO, falls vorhanden, im Bereich von 3% bis 10% (w/w) in Bezug auf das Gesamtgewicht der implantierbaren Vorrichtung liegt.

16. Vorrichtung nach einem der Ansprüche 1-15 zur Verwendung in der Behandlung von HIV-Infektion bei intermittierender Verabreichung in einem Zeitintervall von 2 Wochen bis 3 Monaten.

17. Vorrichtung nach einem der Ansprüche 1-15 zur Verwendung in der Vorbeugung gegen die Übertragung von HIV bei intermittierender Verabreichung in einem Zeitintervall von 2 Wochen bis 3 Monaten.

18. Vorrichtung nach einem der Ansprüche 1-16, wobei Rilpivirin in Basenform verwendet wird.

## Revendications

1. Dispositif implantable en un seul élément comprenant un polymère biocompatible, biodégradable choisi parmi les homopolymères et les copolymères de lactide, glycolide, c-caprolactone, p-dioxanone (1,4-dioxan-2-one) et triméthylène carbonate (1,3-dioxan-2-one) mélangé à la rilpivirine sous forme de base ou d'un sel pharmaceutiquement acceptable ainsi qu'à un ou plusieurs agents améliorant la libération choisis dans le groupe constitué par les poloxamères, les polysorbates et une combinaison de diméthylsulfoxyde (DMSO) et de poly(vinylpyrrolidone)(PVP), où le dispositif contient entre 25 % en masse et 70 % en masse de rilpivirine sous forme de base ou d'un sel pharmaceutiquement acceptable.

2. Dispositif selon la revendication 1, où le dispositif pèse plus de 100 mg.

3. Dispositif selon la revendication 1, où le dispositif pèse plus de 500 mg.

4. Dispositif implantable selon l'une quelconque des revendications 1 à 3, en forme de cylindre.

5. Dispositif implantable selon la revendication 4, ayant un diamètre compris dans l'intervalle de 0,5 mm à 4 mm, et une longueur comprise dans l'intervalle de 1,0 cm à 4 cm.

6. Dispositif implantable selon la revendication 4, ayant un diamètre compris dans l'intervalle de 1,0 mm à 3,0 mm, et une longueur comprise dans l'intervalle de 1,5 cm à 3,5 cm.

7. Dispositif selon l'une quelconque des revendications 1 à 6, où le dispositif contient entre 25 % en masse et 60 % en masse, ou entre 40 % en masse et 60 % en masse, ou entre 50 % en masse et 60 % en masse, de rilpivirine sous forme de base ou d'un sel pharmaceutiquement acceptable.

8. Dispositif selon l'une quelconque des revendications 1 à 7, où le polymère biocompatible, biodégradable est choisi parmi les copolymères de lactide et de glycolide.

9. Dispositif selon la revendication 8, où le polymère biocompatible, biodégradable est un copolymère de lactide et de glycolide dans un rapport molaire de 50 % à 65 % de lactide sur 35 % à 50 % de glycolide.

10. Dispositif selon l'une quelconque des revendications 1 à 9, où l'agent améliorant la libération est un poloxamère.

11. Dispositif selon la revendication 10, où l'agent améliorant la libération est le poloxamère 338.

12. Dispositif selon l'une quelconque des revendications 1 à 11, où le dispositif contient entre 1 % et 40 % en masse par rapport à la masse totale du dispositif implantable, ou entre 10 % et 30 % en masse par rapport à la masse totale du dispositif implantable, ou entre 15 % et 25 % en masse par rapport à la masse totale du dispositif implantable, dudit agent améliorant la libération.

13. Dispositif selon l'une quelconque des revendications 1 à 12, où le dispositif contient entre 10 % et 80 % en masse par rapport à la masse totale du dispositif implantable, ou entre 10 % et 30 % en masse par rapport à la masse totale du dispositif implantable, ou entre 15 % et 25 % en masse par rapport à la masse totale du dispositif implantable, par exemple 20 % en masse par rapport à la masse totale du dispositif implantable dudit polymère biocompatible, biodégradable.

14. Dispositif selon l'une quelconque des revendications 1 à 13, où le dispositif contient entre 15 % et 25 % en masse par rapport à la masse totale du dispositif implantable du polymère biocompatible, biodégradable.

15. Dispositif selon l'une quelconque des revendications 1 à 14, où lorsque le DMSO est présent, sa teneur est comprise dans l'intervalle allant de 3 % à 10 % en masse par rapport à la masse totale du dispositif implantable.

16. Dispositif selon l'une quelconque des revendications 1 à 15, pour utilisation dans le traitement d'une infection par le VIH en cas d'administration intermittente dans un intervalle de temps de 2 semaines à 3 mois.

17. Dispositif selon l'une quelconque des revendications 1 à 15, pour utilisation dans la prévention d'une transmission du VIH en cas d'administration intermittente dans un intervalle de temps de 2 semaines à 3 mois.

18. Dispositif selon l'une quelconque des revendications 1 à 16, où la rilpivirine est utilisée sous forme de base.
